(19) Europäisches Patentamt
European Patent Office
Office européen des brevets

(11) Veröffentlichungsnummer: **0 118 082**
**B1**

(12) # EUROPÄISCHE PATENTSCHRIFT

(45) Veröffentlichungstag der Patentschrift: **18.12.85**

(21) Anmeldenummer: **84101877.3**

(22) Anmeldetag: **23.02.84**

(51) Int. Cl.⁴: **C 07 C 43/225**, C 07 C 43/215, C 07 C 43/285, C 07 C 79/355, C 07 C 121/75, C 07 C 41/16, C 07 C 41/24

(54) **Phenoxyacetylene und Verfahren zu ihrer Herstellung.**

(30) Priorität: **04.03.83 DE 3307636**

(43) Veröffentlichungstag der Anmeldung: **12.09.84 Patentblatt 84/37**

(45) Bekanntmachung des Hinweises auf die Patenterteilung: **18.12.85 Patentblatt 85/51**

(84) Benannte Vertragsstaaten: **AT BE CH DE FR GB IT LI NL SE**

(56) Entgegenhaltungen:
**EP - A - 0 056 933**
**GB - A - 617 820**

**TETRAHEDRON LETTERS, Nr. 34, 1978, Seiten 3103-3106, Pergamon Press Ltd., GB; K. TANAKA et al.: "New applications of organofluorine reagents in organic synthesis. III. A convenient synthetic method for acetylenic ethers and thioethers"**
**CHEMICAL ABSTRACTS, Band 71, Nr. 15, 13. Oktober 1969, Seite 276, Nr. 70234u, Columbus, Ohio, USA; M.F. SHOSTAKOVSKII et al.: "Synthesis of aryloxyacetylenes and their beta-methyl derivatives"**
**CHEMICAL ABSTRACTS, Band 80, Nr. 21, 27. Mai 1974, Seite 394, Nr. 120434t, Columbus, Ohio, USA; A.KH. FILIPPOVA et al.: "Prototropic acetylene-allene isomerization of aroxy-alkynes"**

(73) Patentinhaber: **BASF Aktiengesellschaft, Carl-Bosch-Strasse 38, D-6700 Ludwigshafen (DE)**

(72) Erfinder: **Ruland, Alfred, Dr., Schriesheimer Strasse 11, D-6945 Hirschberg (DE)**
Erfinder: **Reuther, Wolfgang, Dr., Am Pferchelhang 16, D-6900 Heidelberg (DE)**

(56) Entgegenhaltungen: (Fortsetzung)
**JOURNAL OF THE AMERICAN CHEMICAL SOCIETY, Band 62, Nr. 7, 6. Juli 1940, Seiten 1849-1854, T.L. JACOBS et al.: "Acetylenic ethers. I. Phenoxyacetylenes"**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die vorliegende Erfindung betrifft neue alkyl-substituierte Phenoxyacetylene und ein Verfahren zur Herstellung von alkyl-substituierten Phenoxyacetylenen durch Umsetzung von 1,1-Dihalogenolefinen mit Phenolaten in Gegenwart eines Katalysators zu α-Halogenolethern und Eliminierung von Halogenwasserstoff aus diesen Verbindungen. Die neuen alkyl-substituierten Phenoxyacetylene sind wertvolle Zwischenprodukte zur Herstellung von fungizid wirksamen Azol-Derivaten, z. B. alkyl-substituierte Keten-triazolylphenoxylderivaten (DE-OS Nr. 3100261.7).

Zur Synthese von alkyl-substituierten, phenolischen Acetylenethern ist im Prinzip folgendes bekannte Verfahren anwendbar:

Halogenierung von 1-Phenoxy-1-alkenen zu 1-Phenoxy-1,2-dihalogenalkanen und anschliessende zweifache Eliminierung von Halogenwasserstoff zu den entsprechenden Acetylenethern (L. Brandsma, H.J.T. Bos, J.F. Arens in Chemistry of Acetylenes, Verlag Dekker, S. 756 ff.). Dieses Verfahren hat jedoch einige Nachteile. Zum einen sind die als Ausgangsstoffe benötigten 1-Phenoxy-1-alkene selbst nur schlecht durch zum Teil mehrstufige Synthesen aus 1-Phenoxyalkylketonen, 1-Phenoxy-2-halogenalkanen oder 1-Phenoxy-1-halogenalkanen zugänglich, die ihrerseits nur schlecht über mehrstuffige Verfahren zugänglich sind und somit in technischem Massstab nur schwierig hergestellt werden können. Zum anderen entstehen 1-Phenoxy-1,2-dihalogenalkane in der Regel als Diastereomerengemische, die beim ersten Eliminierungsschritt der Halogenwasserstoffabspaltung Gemische E/Z-isomerer 1-Phenoxy-2-halogen-1-alkene liefern, von denen nur die Z-Isomeren durch weitere Halogenwasserstoffabspaltung glatt in die entsprechenden Phenoxyacetylene überführbar sind. Das bedeutet neben einer Ausbeuteverminderung zusätzliche Reinigungsverfahren zur Abtrennung des Endproduktes von nicht umgesetztem Ausgangsprodukt.

Es wurde nun gefunden, dass die neuen alkyl-substituierten Phenoxyacetylene der allgemeinen Formel

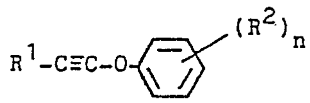

I,

in welcher $R^1$ Propyl, tert.-Butyl oder Hexyl und $R^2$ Halogen, insbesondere 2,4-Dichlor, Alkyl mit 1 bis 4 C-Atomen, Alkoxy mit 1 bis 4 C-Atomen, Cyano, Nitro, Trifluormethyl oder Aryl, insbesondere Phenyl, oder Phenoxy bedeutet, wobei der Phenyl- oder Phenoxyrest selbst wieder substituiert durch Halogen oder Alkyl mit 1 bis 4 C-Atomen sein kann und n eine ganze Zahl von 1 bis 5 ist, wertvoll Zwischenprodukte für die Herstellung von fungiziden Azolderivaten sind.

Halogen bedeutet Fluor, Chlor, Brom oder Iod.

Alkylreste mit 1 bis 4 C-Atomen sind beispielsweise Methyl, Ethyl, Propyl, Butyl.

Alkoxyreste mit 1 bis 4 C-Atomen sind z. B. Methoxy oder Ethoxy.

Es wurde ferner gefunden, das alkyl-substituierte Phenoxyacetylene der Formel

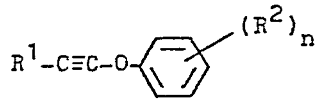

I,

in der $R^1$ einen Alkylrest mit 1 bis 8 C-Atomen, $R^2$ Halogen, Alkyl mit 1 bis 4 C-Atomen, Alkoxy mit 1 bis 4 C-Atomen, Cyano, Nitro, Trifluormethyl oder Phenyl oder Phenoxy bedeutet, wobei der Phenyl- oder Phenoxyrest selbst wieder substituiert sein kann durch Halogen oder Alkyl mit 1 bis 4 C-Atomen und n = 0 oder eine ganze Zahl von 1 bis 5 ist, in einfacher Weise und guten Ausbeuten erhalten werden können, indem man

a) ein 1,1-Dihalogenolefin der Formel II

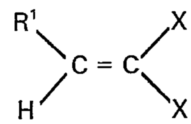

II,

in der $R^1$ die oben genannte Bedeutung hat und X Halogen bedeutet, mit einem Alkali-, Erdalkali- oder Aluminiumphenolat der Formel

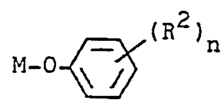

in der $R^2$ und n die oben genannten Bedeutungen haben und M ein Äquivalent eines Alkali-, Erdalkali- oder Aluminiumatoms bedeutet, in Gegenwart eines Kupfersalzes oder einer Kupferverbindung und eines polaren Lösungsmittels bei einer Temperatur von 50 bis 250° C zu einem Halogenenolether der Formel III umsetzt

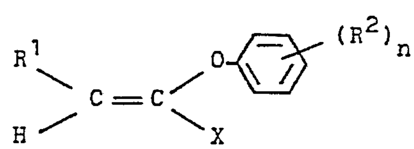

III,

in der $R^1$, $R^2$, X und n die oben genannten Bedeutungen haben, und

b) die Verbindung der Formel III in Gegenwart einer Base und eines Lösungsmittels bei einer Temperatur von −40 bis +100° C unter Halogenwasserstoffabspaltung zu dem alkyl-substituierten Phenoxyacetylen der Formel

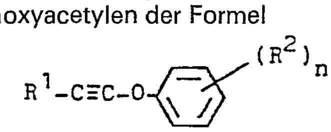

I,

umsetzt, in der $R^1$, $R^2$ und n die oben genannten Bedeutungen haben.

Alkali ist beispielsweise Natrium oder Kalium.

Erdalkali ist beispielsweise Magnesium, Calcium oder Strontium.

Die Temperatur für die Umsetzung a liegt im Bereich von vorzusgsweise 140 bis 200° C.

Als Katalysatoren finden Kupfersalze, z. B. CuCl, CuBr, Kupfer-(I)-iodid, $CuCl_2$, $CuBr_2$, Kupfer-(II)-iodid, CuCN, $Cu(CN)_2$, Kupfer-(II)-acetat oder Kupferverbindungen, z. B. Kupfer-(II)-hydroxid, Verwendung, wobei das Kupfer-(II)-hydroxid bevorzugt wird. Als Lösungsmittel können polare Lösungsmittel wie Alkohole, z. B. Methanol, Ethanol, ferner Ketone wie Aceton, Methylethylketon, ferner Dimethylformamid, Dimethylsulfoxid, Hexamethylphosphorsäuretriamid und N-Methylpyrrolidon angewendet werden. Auch Mischungen aus organischen Lösungsmitteln und Wasser können Verwendung finden.

Die Entstehung der alkyl-substituierten 1-Phenoxy-1-halogen-1-alkene unter den oben beschriebenen Reaktionsbedingungen ist um so überraschender, als bei entsprechenden Umsetzungen ohne Katalysator bisher ausschliesslich die isomeren 1-Halogen-2-phenoxy-1-alkene erhalten wurden, die für eine weitere Umsetzung zu den Acetylenderivaten der Formel I nicht geeignet sind (DE-OS Nr. 3101143).

Die nach dem oben beschriebenen Verfahren erhaltenen 1-Phenoxy-1-halogen-1-alkene der Formel III werden entweder direkt ohne Abtrennung aus der reaktionsmischung z. B. im Eintropfverfahren oder nach Abtrennung von der Reaktionsmischung und evtl. Reinigung zu den Acetylenderivaten der Formel I in Gegenwart von Basen umgesetzt.

Verwendet man beispielsweise 1-(2,4-Dichlorphenoxy)-1-chlor-3,3,-dimethylbut-1-en als Ausgangsverbindung, so kann der Reaktionsablauf zum 1-(2,4-Dichlorphenoxy)-3,3-dimethylbut-1-in durch das folgende Formelschema wiedergegeben werden:

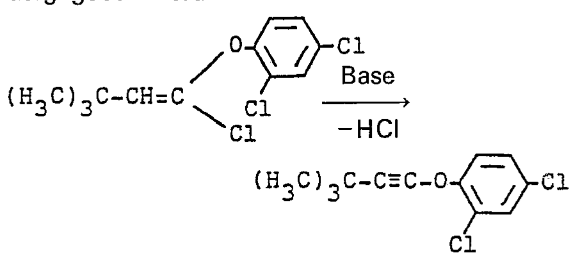


Als basische Stoffe für die Eliminierung von Halogenwasserstoff gemäss b kommen sowohl anorganische als auch organische Basen in Betracht. Vorzugsweise werden Alkali- und Erdalkali-Hydroxide, -Carbonate oder -Alkoholate, z. B. Natriummethylat oder Kalium-tert.-butanolat, eingesetzt.

Geeignete Lösungsmittel für b sind Alkohole wie Methanol, Ethanol und tert.-Butanol, Ether wie Tetrahydrofuran und Dioxan, ferner Ketone wie Aceton und Methylisobutylketon, ferner Acetonitril, Dimethylformamid, Dimethylsulfoxid, N-Methylpyrrolidon und Hexamethylphosphorsäuretriamid. Auch die Verwendung von Zweiphasensystemen (organisches Lösungsmittel/Wasser) unter Einsatz von Phasentransferkatalysatoren, z. B. Ammonium- und Phosphoniumsalzen, ist möglich.

Die Eliminierungsreaktion b wird beispielsweise bei Temperaturen zwischen −10 und +40° C durchgeführt.

Das neue Verfahren hat folgende Vorteile:

Die als Ausgangsverbindungen benötigten Dihalogenolefine der Formel II sind leicht und preiswert aus einfache Rohstoffen wie Alkylhalogeniden bzw. Alkenen und HCl (z. B. Isobutylen und HCl) sowie 1,1-Dihalogenethylenen durch Aluminiumchlorid-Katalyse nach bekannten Verfahren zu erhalten. Davon ausgehend sind die Phenoxyacetylene der Formel I in max. zwei Stufen zugänglich, wobei die Zwischenverbindungen der Formel III in der Regel als Z-Isomere anfallen, aus denen Halogenwasserstoff leicht quantitativ eliminierbar ist.

Folgende Beispiele sollen das Verfahren näher erläutern:

*Beispiel 1*

*Herstellung von 1-(2,4-Dichlorphenoxy)-3,3-dimethylbut-1-in*

a) *Herstellung der Zwischenverbindung 1-Chlor-1-(2,4-dichlorphenoxy)-3,3-dimethylbut-1-en*

153 g (1 mol) 1,1-Dichlor-3,3-dimethylbut-1-en (erhalten durch Umsetzung von tert.-Butylchlorid mit Vinylidenchlorid in Gegenwart von Eisen-(III)-chlorid), 163 g (1 mol) 2,4-Dichlorphenol, 51,2 g (1 mol) Natriummethylat und 2 g Kupfer-(II)-hydroxid werden unter $N_2$ in 500 ml Ethanol 6 h im Autoklaven auf 180° C erhitzt. Man filtriert vom ausgefallenen Salz ab, destilliert im Vakuum zunächst das Lösungsmittel ab und fraktioniert anschliessend das zurückbleibende Rohprodukt.

Sdp.: 90 bis 95° C bei 0,01 mbar

Ausbeute: 139,7 g (50% d. Th.).

$^1$H-NMR (250 MHz, δ-Werte in ppm, LM: D-DMSO)

δ = 1,2 (9 H); 5,75 (s, 1 H); 7,18 (d, 1 H, Jo = Hz); (dd, 1 H, Jo = 10 Hz, Jm = 2,5 Hz); 7,7 (d, 1 H, Jm = 2,5 Hz).

b) *Eliminierung von Chlorwasserstoff*

112 g (0,4 mol) 1-Chlor-1-(2,4-dichlorphenoxy)-3,3-dimethylbut-1-en werden bei 0° C in 500 ml Tetrahydrofuran mit 45 g (0,4 mol) K-tert.-butanolat versetzt und anschliessend 1 h bei Raumtemperatur (20° C) gerührt. Danach gibt man das gleiche Volumen Wasser zu, extrahiert mit Ether und destilliert das Lösungsmittel im Vakuum ab. Das so erhaltene Rohprodukt wird aus Methanol umkristallisiert.

Ausbeute: 87,4 g (90% d. Th.)

Schmp.: 34° C

$^1$H-NMR (250 MHz, δ-Werte in ppm, LM: D-DMSO)

δ = 1,25 (s, 9 H); 7,56 (s, 1 H, Jo ~4 Hz); 7,565 (d, 1 H, Jm <1 Hz); 7,73 (dd, 1 H, Jo ~4 Hz, Jm <1 Hz).

**Patentansprüche** für die Vertragsstaaten BE, CH, DE, FR, GB, IT, LI, NL, SE

1. Phenoxyacetylen der allgemeinen Formel

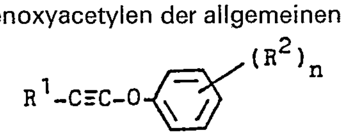

(I)

in der R¹ Propyl, tert.-Butyl oder Hexyl, R² Halogen, Alkyl mit 1 bis 4 C-Atomen, Alkoxy mit 1 bis 4 C-Atomen, Cyano, Nitro, Trifluormethyl oder Phenyl oder Phenoxy bedeutet, wobei der Phenyl- oder Phenoxyrest selbst wieder substituiert sein kann durch Halogen oder Alkyl mit 1 bis 4 C-Atomen und n eine ganze Zahl von 1 bis 5 ist.

2. Verfahren zur Herstellung eines alkyl-substituierten Phenoxyacetylens der Formel

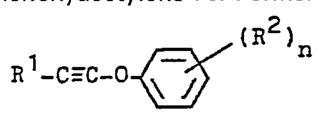
(I)

in der $R^1$ einen Alkylrest mit 1 bis 8 C-Atomen, $R^2$ Halogen, Alkyl mit 1 bis 4 C-Atomen, Alkoxy mit 1 bis 4 C-Atomen, Cyano, Nitro, Trifluormethyl oder Phenyl oder Phenoxy bedeutet, wobei der Phenyl- oder Phenoxyrest selbst wieder substituiert sein kann durch Halogen oder Alkyl mit 1 bis 4 C-Atomen und n = 0 oder eine ganze Zahl von 1 bis 5 ist, dadurch gekennzeichnet, dass man

a) ein 1,1-Dihalogenolefin der Formel (II)

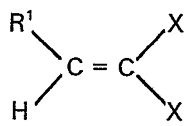
(II)

in der $R^1$ die oben genannte Bedeutung hat und X halogen bedeutet, mit einem Alkali-, Erdalkali- oder Aluminiumphenolat der Formel

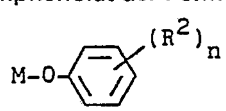

in der $R^2$ und n die oben genannten Bedeutungen haben und M ein Äquivalent eines Alkali-, Erdalkali- oder Aluminiumatoms bedeutet, in Gegenwart eines Kupfersalzes oder einer Kupferverbindung und eines polaren Lösungsmittels bei einer Temperatur von 50 bis 250° C zu einem α-Halogenenolether der Formel (III) umsetzt

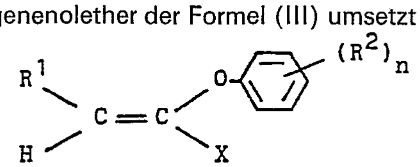
(III)

in der $R^1$, $R^2$, X und n die oben genannten Bedeutungen haben, und

b) die Verbindung der Formel (III) in Gegenwart einer Base und eines Lösungsmittels bei einer Temperatur von −40 bis +100° C unter Halogenwasserstoffabspaltung zu dem alkyl-substituierten Phenoxyacetylen der Formel

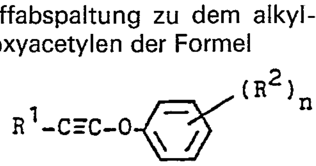
(I)

umsetzt, in der $R^1$, $R^2$ und n die oben genannten Bedeutungen haben.

3. 1-(2,4-Dichlorphenoxy)-3,3-dimethylbut-1-in gemäss Anspruch 1.

**Patentansprüche** für den Vertragsstaat: AT

1. Verfahren zur Herstellung eines alkyl-substituierten Phenoxyacetylens der Formel

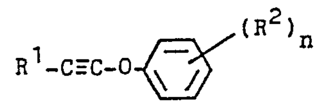
(I)

in der $R^1$ einen Alkylrest mit 1 bis 8 C-Atomen, $R^2$ Halogen, Alkyl mit 1 bis 4 C-Atomen, Alkoxy mit 1 bis 4 C-Atomen, Cyano, Nitro, Trifluormethyl oder Phenyl oder Phenoxy bedeutet, wobei der Phenyl- oder Phenoxyrest selbst wieder substituiert sein kann durch Halogen oder Alkyl mit 1 bis 4 C-Atomen und n = 0 oder eine ganze Zahl von 1 bis 5 ist, dadurch gekennzeichnet, dass man

a) ein 1,1-Dihalogenolefin der Formel (II)

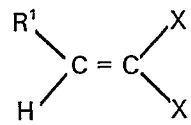
(II)

in der $R^1$ die oben genannte Bedeutung hat und X Halogen bedeutet, it einem Alkali-, Erdalkali- oder Aluminiumphenolat der Formel

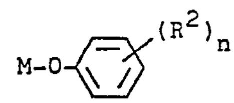

in der $R^2$ und n die oben genannten Bedeutungen haben und M ein Äquivalent eines Alkali, Erdalkali- oder Aluminiumatoms bedeutet, mit Gegenwart eines Kupfersalzes oder einer Kupferverbindung und eines polaren Lösungsmittels bei einer Temperatur von 50 bis 250° C zu einem α-Halogenenolether der Formel (III) umsetzt

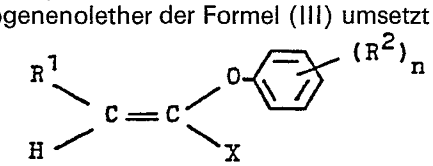
(III)

in der $R^1$, $R^2$, X und n die oben genannten Bedeutungen haben, und

b) die Verbindung der Formel (III) in Gegenwart einer Base und eines Lösungsmittels bei einer Temperatur von −40 bis +100° C unter Halogenwasserstoffabspaltung zu einem alkyl-substituierten Phenoxyacetylen der Formel

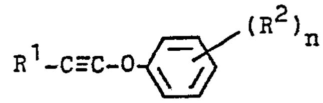

umsetzt, in der $R^1$, $R^2$ und n die oben genannten Bedeutungen haben.

2. Verfahren nach Anspruch 1, dadurch gekennzeichnet, dass man durch Umsetzung von 1,1-Dichlor-3,3-dimethylbut-1-en mit 2,4-Dichlorphenol die Verbindung 1-(2,4-Dichlorphenoxy)-3,3-dimethylbut-1-in herstellt.

**Claims** for the Contracting States BE, CH, DE, FR, GB, IT, LI, NL, SE

1. A phenoxyacetylene of the general formula

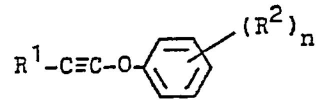
(I)

where $R^1$ ist propyl, tert.-butyl or hexyl, $R^2$ is halogen, alkyl of 1 to 4 carbon atoms, alkoxy of 1 to 4 carbon atoms, cyano, nitro, trifluoromethyl, phenyl or phenoxy, it being possible for the phenyl or phenoxy radical itself to be also substituted by halogen or alkyl of 1 to 4 carbon atoms, and n is an integer from 1 to 5.

2. A process for the preparation of an alkylsubstituted phenoxyacetylene of the formula

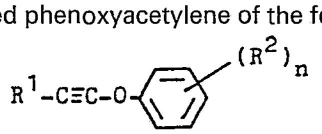 (I)

where $R^1$ is alkyl of 1 to 8 carbon atoms, $R^2$ is halogen, alkyl of 1 to 4 carbon atoms, alkoxy of 1 to 4 carbon atoms, cyano, nitro, trifluoromethyl, phenyl or phenoxy, it being possible for the phenyl or phenoxy radical itself to be also substituted by halogen or alkyl of 1 to 4 carbon atoms, and n is 0 or an integer from 1 to 5, wherein

(*a*) a 1,1-dihalo-olefin of the Formula (II)

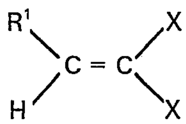 (II)

where $R^1$ has the above meaning and X is halogen, is reacted with an alkali metal, alkaline earth metal or aluminium phenolate of the formula

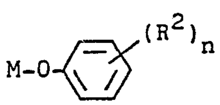

where $R^2$ and n have the above meanings and M is one equivalent of an alkali metal, alkaline earth metal or aluminium atom, in the presence of a copper salt or a copper compound and a polar solvent at a temperature of from 50 to 250° C to give an α-halo-enol ether of the Formula (III)

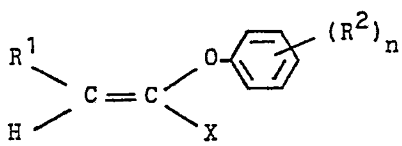 (III)

where $R^1$, $R^2$, X and n have the above meanings, and

(*b*) the compoud of the Formula (III) is reacted in the presences of a base and a solvent at a temperature of from −40 to +100° C, with the elimination of hydrogen halide, to give the alkylsubstituted phenoxyacetylene of the formula

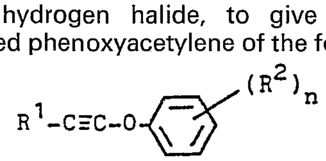 (I)

where $R^1$, $R^2$ and n have the above meanings.

3. 1.-(2,4-Dichlorophenoxy)-3,3-dimehtylbut-1-yne according to Claim 1.

**Claims** for the Contracting State: AT

1. A process for the preparation of an alkylsubstituted phenoxyacetylene of the formula

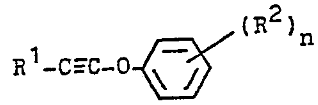 (I)

where $R^1$ is alkyl of 1 to 8 carbon atoms, $R^2$ is halogen, alkyl of 1 to 4 carbon atoms, alkoxy of 1 to 4 carbon atoms, cyano, nitro, trifluoromethyl, phenyl or phenoxy, it being possible for the phenyl or phenoxy radical itself to be also substituted by halogen or alkyl of 1 to 4 carbon atoms, and n is 0 or an integer from 1 to 5, wherein

(*a*) a 1,1-dihalo-olefin of the Formula (II)

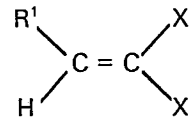 (II)

where $R^1$ has the above meaning and X is halogen, is reacted with an alkali metal, alkaline earth metal or aluminium phenolate of the formula

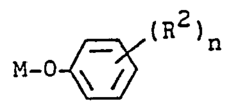

where $R^2$ and n have the above meanings ans M is one equivalent of an alkali metal, alkaline earth metal or aluminium atom, in the presence of a copper salt or a copper compound and a polar solvent at a temperature of from 50 to 250° C to give an α-halo-enol ether of the Formula (III)

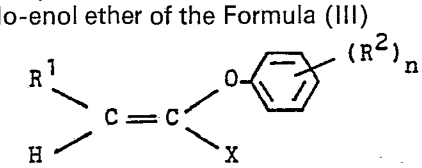 (III)

where $R^1$, $R^2$, X and n have the above meanings, and

(*b*) the compound of the Formula (III) is reacted in the presence of a base and a solvent at a temperature of from −40 to +100° C, with the elimination of hydrogen halide, to give the alkylsubstituted phenoxyacetylene of the formula

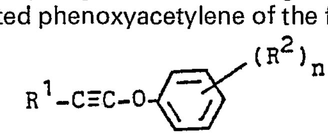

where $R^1$, $R^2$ and n have the above meanings.

2. A process as claimed in Claim 1, wherein the compound 1-(2,4-dichlorophenoxy)-3,3-dimethylbut-1-yne is prepared by reacting 1,1-dichloro-3,3-dimethylbut-1-ene with 2,4-dichlorophenol.

**Revendications** pour les Etats contractants BE, CH, DE, FR, GB, IT, LI, NL, SE

1. Phénoxyacétylène de formule générale

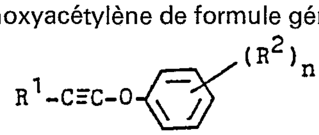 (I)

dans laquelle $R^1$ représente propyle, tert.-butyle ou hexyle,

$R^2$ représente halogène, alkyle ayant 1 à

4 atomes C, alcoxy ayant 1 à 4 atomes C, cyano, nitro, trifluorométhyle ou phényle ou phénoxy, le reste phényle ou phénoxy pouvant, à son tour, être substitué par halogène ou alkyle de 1 à 4 atomes C, et n est un nombre entier de 1 à 5.

2. Procédé de préparation d'un phénoxyacétylène de formule

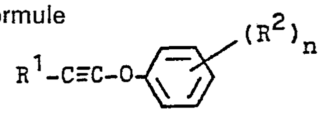
(I)

dans laquelle $R^1$ représente un reste alkyle ayant 1 à 8 atomes C,

$R^2$ représente halogène, alkyle ayant 1 à 4 atomes C, alcoxy ayant 1 à 4 atomes C, cyano, nitro, trifluorométhyle, phényle ou phénoxy, le reste phényle ou phénoxy pouvant, à son tour, être substitué par halogène ou alkyle de 1 à 4 atomes C, et n = 0 ou un nombre entier de 1 à 5, ce procédé étant caractérisé par le fait que

a) on fait réagir une 1,1-dihalogène-oléfine de formule (II)

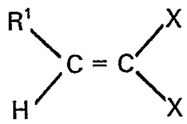
(II)

dans laquelle $R^1$ a la signification donnée plus haut et X représente halogène, avec un phénolate alcalin, alcalino-terreux ou d'aluminium, de formule

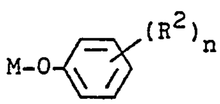

dans laquelle $R^2$ et n ont les significations données plus haut, M étant l'équivalent d'un atome alcalin, alcalino-terreux ou d'aluminium, en présence d'un sel de cuivre ou d'un composé de cuivre et d'un solvant polaire, à une température de 50 à 250° C, pour obtenir un éther α-halogène-énolique de formule (III)

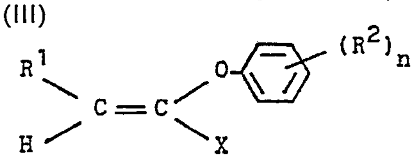
(III)

dans laquelle $R^1$, $R^2$, X et n ont les significations données plus haut, et

b) on transforme le composé de formule (III), en présence d'une base et d'un solvant, à une température de −40 à +100° C, avec séparation d'hydracide, en phénoxyacétylène substitué par alkyle, de formule

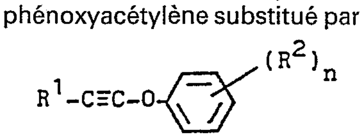
(I)

dans laquelle $R^1$, $R^2$ et n ont les significations données plus haut.

3. 1-(2,4-dichlorophénoxy)-3,3-diméthylbut-1-yne selon la revendication 1.

**Revendications** pour l'Etat contractant: AT

1. Procédé de préparation d'un phénoxyacétylène de formule

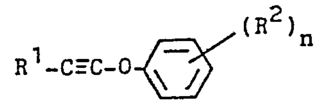
(I)

dans laquelle $R^1$ représente un reste alkyle ayant 1 à 8 atomes C,

$R^2$ représente halogène, alkyle ayant 1 à 4 atomes C, alcoxy ayant 1 à 4 atomes C, cyano, nitro, trifluorométhyle, phényle ou phénoxy, le reste phényle ou phénoxy pouvant, à son tour, être susbtitué par halogène ou alkyle de 1 à 4 atomes C, et n = 0 ou un nombre entier de 1 à 5, ce procédé étant caractérisé par le fait que:

a) on fait réagir une 1,1-dihalogène-oléfine de formule (II)

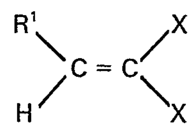
(II)

dans laquelle $R^1$ représente propyle, tert.-butyle ou hexyle, et X représente halogène, avec un phénolate alcalin, alcalino-terreux ou d'aluminium, de formule

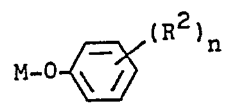

dans laquelle $R^2$ représente halogène, alkyle ayant 1 à 4 atomes C, alcoxy ayant 1 à 4 atomes C, cyano, nitro, trifluorométhyle ou phényle ou phénoxy, le reste phényle ou phénoxy pouvant, à son tour, être substitué par halogène ou alkyle de 1 à 4 atomes C, et n est un nombre entier de 1 à 5, M étant l'équivalent d'un atome alcalin, alcalino-terreux ou d'aluminium, en présence d'un sel de cuivre ou d'un composé de cuivre et d'un solvant polaire, à une température de 50 à 250° C, pour obtenir un éther α-halogène-énolique de formule (III)

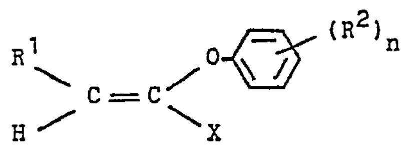
(III)

dans laquelle $R^1$, $R^2$, X et n ont les significations données plus haut, et

b) on transforme le composé de formule (III), en présence d'une base et d'un solvant, à une température de −40 à +100° C, avec séparation d'hydracide, en phénoxyacétylène substitué par alkyle, de formule

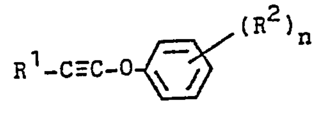
(I)

dans laquelle $R^1$, $R^2$ et n ont les significations données plus haut.

2. Procédé selon la revendication 1, caractérisé par le fait qu'on prépare le composé 1-(2,4-dichlorophénoxy)-3,3-diméthylbut-1-yne en faisant réagir du 1,1-dichloro-3,3-diméthylbut-1-ène avec du 2,4-dichlorophénol.